# FASCICULE DE BREVET EUROPEEN

(11) **EP 2 349 959 B1**
(45) Date de publication et mention de la délivrance du brevet: **23.07.2014**
(21) Numéro de dépôt: 09740479.2
(22) Date de dépôt: 20.08.2009
(51) Int. Cl.: C07C 17/23, C07C 21/18

(54) **PROCEDE DE PREPARATION DU FLUORURE DE VINYLIDENE**
VERFAHREN ZUR HERSTELLUNG VON VINYLIDENFLUORID
METHOD FOR PREPARING VINYLIDENE FLUORIDE

(30) Priorité: 13.10.2008 FR 0856898
(43) Date de publication de la demande: 03.08.2011
(73) Titulaire: Arkema France, 92700 Colombes (FR)
(72) Inventeur: PERDRIEUX, Sylvain, F-69390 Charly (FR); HUB, Serge, F-69100 Villeurbanne (FR)
(86) Numéro de dépôt international: PCT/FR2009/051611
(87) Numéro de publication internationale: WO 2010/043792

(56) Documents cités:
- EP-A- 0 485 246
- WO-A-90/08748
- GB-A- 2 165 241
- US-A- 2 774 798
- US-A- 2 774 799
- US-A- 3 183 277

## Description

La présente invention concerne le domaine des hydrocarbures fluorés insaturés et a plus particulièrement pour objet la synthèse du fluorure de vinylidène (VF₂) à partir du 1,2-dichloro-2,2-difluoroéthane (F132b).

Les oléfines fluorées, comme le VF₂, sont connues et sont utilisées comme monomères ou co-monomères pour la fabrication de polymères fluorocarbonés présentant des caractéristiques remarquables, en particulier une excellente tenue chimique et une bonne résistance thermique.

Le VF₂ peut être fabriqué par déshydrohalogénation, soit du 1,1,1-chlorodifluoroéthane (F142b) par perte d'HCl, soit du 1,1,1-trifluoroéthane (F143a) par perte d'HF. Les procédés mis en oeuvre ont l'inconvénient d'utiliser comme réactifs des entités issues de l'hydrofluoration totale ou partielle du 1,1,1-trichloroéthane, dont l'accès est limité.

Le VF₂ peut enfin être obtenu à partir du 1-chloro-2,2-difluoroéthane (F142) provenant de l'hydrofluoration partielle du 1,1,2-trichloroéthane dont la disponibilité est plus aisée. Ainsi, le document US 2 774 799 décrit un procédé d'obtention de fluorure de vinylidène en chauffant le difluorochloroéthane à une température comprise entre 500 et 730°C et un temps de contact compris entre 1 et 60 secondes en présence du cuivre métallique et de ses alliages.

Le VF₂ peut aussi être fabriqué par déshydrohalogénation catalytique du 1,2-dichloro-2,2-difluoroéthane (F132b) en présence d'hydrogène par perte de deux molécules d'HCl. L'exemple 4 du document EP485246 décrit l'utilisation d'un catalyseur mixte de palladium et de cuivre déposé sur du charbon. On peut aussi utiliser des amalgames alcalins, comme le mentionne le brevet GB2165241 mais alors le procédé génère du sel, qu'il faut traiter, éliminer ou valoriser.

Les procédés catalytiques présentent de nombreux inconvénients tels que la préparation du catalyseur, l'activation et le démarrage d'un catalyseur neuf, la désactivation du catalyseur, le risque de bouchage du réacteur chargé

La présente invention fournit un procédé d'obtention du fluorure de vinylidène en l'absence de catalyseur. Plus particulièrement, le procédé selon la présente invention consiste en la pyrolyse du 1,2-dichloro-2,2-difluoroéthane en présence d'hydrogène à une température supérieure ou égale à 400°C pour donner du fluorure de vinylidène. De préférence la température est comprise entre 400 et 1000°C ; avantageusement comprise entre 450 et 750°C et plus particulièrement à une température comprise entre 500 et 600°C.

On entend par pyrolyse, une transformation chimique sous l'effet de la température en l'absence de catalyseur.

Les réacteurs de pyrolyse comprennent en général trois zones : (a) la zone de préchauffage dans laquelle les réactifs sont mis en contact à une température proche de celle de la réaction, (b) la zone de réaction dans laquelle les réactifs sont à la température de réaction et sont au moins partiellement transformés en produits et sous-produits et (c) une zone de trempe dans laquelle le flux issu de la zone réactionnelle est refroidi pour arrêter la réaction de pyrolyse. A l'échelle laboratoire, les réacteurs ne peuvent comprendre que la zone réactionnelle; on peut ignorer les zones de préchauffage et de trempe.

Selon la présente invention, la réaction de pyrolyse du 1,2-dichloro-2,2 difluoroéthane en présence d'hydrogène est effectuée en l'absence de catalyseur dans un réacteur essentiellement vide. On entend par absence de catalyseur le fait qu'il n'y a pas eu ajout de matériau ou de traitement dans le réacteur pour augmenter la vitesse de réaction par réduction de l'énergie d'activation du procédé:

Plus particulièrement, on entend par absence de catalyseur, absence de catalyseur conventionnel ayant une surface spécifique et se présentant sous forme de particules, d'extrudés, supporté ou non pour faciliter la réaction de déhydrochloration.

Les réacteurs essentiellement vides qui conviennent sont des réacteurs à quartz, en céramique (SiC) ou métalliques. Dans ce cas, le matériau constituant le réacteur peut être choisi parmi les métaux tels que le nickel, le fer, le titane, le chrome, le molybdène, le cobalt, l'or ou leurs alliages. Le métal,

Les réacteurs essentiellement vides qui conviennent sont des réacteurs à quartz, en céramique (SiC) ou métalliques. Dans ce cas, le matériau constituant le réacteur peut être choisi parmi les métaux tels que le nickel, le fer, le titane, le chrome, le molybdène, le cobalt, l'or ou leurs alliages. Le métal, choisi plus particulièrement pour limiter la corrosion ou les phénomènes catalytiques, peut être massif ou plaqué sur un autre métal.

Les réacteurs essentiellement vides utilisables dans la présente invention peuvent comprendre des chicanes ou des garnissages inertes tels que les anneaux Raschig pour faciliter le mélange gazeux.

Le rapport molaire hydrogène / F132b peut être compris entre 0,2 et 20, de préférence compris entre 0,5 et 10 et avantageusement entre 1 et 6.

Outre les réactifs, la réaction peut être mise en oeuvre en présence d'un gaz inerte tel que l'azote, l'hélium et l'argon.

Le temps de contact, défini comme étant le rapport entre le volume du réacteur vide et le débit volumique du flux gazeux dans les conditions normales de température et de pression, peut être compris entre 0,1 et 500 secondes et de préférence compris entre 0,5 et 100 secondes et avantageusement compris entre 1 et 50 secondes.

Industriellement, on préfère travailler à la pression atmosphérique, mais on ne sortirait pas du cadre de la présente invention en travaillant à une pression inférieure ou supérieure à la pression atmosphérique, pourvu que le système réactionnel reste à l'état gazeux.

Les exemples suivants illustrent l'invention sans la limiter.

### EXEMPLE 1

Dans un réacteur tubulaire en inox de 45 cm de long et de 10 mm de diamètre intérieur, chauffé électriquement, on introduit un mélange d'hydrogène et de F132b dans les conditions suivantes :
Pression : atmosphérique
Température : 550°C
Débit de F132b : 0,047 mole/h
Débit d'hydrogène : 0,094 mole/h
Ratio H₂/F132b : 2 molaire
Temps de séjour : 13,4 s

L'analyse est effectuée par chromatographie en phase gazeuse (CPG) sur les gaz issus du réacteur. L'identification des produits a été confirmée par spectrométrie de masse.

Le produit majeur de la réaction est du VF₂. Le taux de conversion du F132b est de 84 % et la sélectivité en VF₂ est de 86 %.

Le taux de conversion est le pourcentage du produit de départ ayant réagi (nombre de mole du produit de départ ayant réagi / nombre de mole du produit de départ introduit).

La sélectivité en produit recherché est le ratio nombre de mole du produit recherché / nombre de mole du produit de départ ayant réagi.

### EXEMPLE 2

On opère comme à l'exemple 1 sauf que le débit du F132b est de 0,094 mole/h et le débit de l'hydrogène est de 0,375 mole/h et le ratio molaire hydrogène / F132b est de 4.

Le temps de contact est de 4 sec.

Le taux de conversion du F132b est de 73 % et la sélectivité en VF₂ est de 87 %.

### EXEMPLE 3

On opère comme à l'exemple 2, sauf que la température est de 600°C.

Le taux de conversion du F132b est de 97 % et la sélectivité en VF₂ est de 82 %.

## Revendications

1. Procédé d'obtention du fluorure de vinylidène consistant en la pyrolyse du 1,2-dichloro-2,2-difluoroéthane en présence d'hydrogène et en l'absence de catalyseur à une température supérieure ou égale à 400°C.

2. Procédé selon la revendication 1 **caractérisé en ce que** la température est comprise entre 400 et 1000°C.

3. Procédé selon la revendication 1 ou 2 **caractérisé en ce que** la température est comprise entre 450 et 750°C.

4. Procédé selon l'une quelconque des revendications précédentes **caractérisé en ce que** la température est comprise entre 500 et 600°C.

5. Procédé selon l'une quelconque des revendications précédentes **caractérisé en ce que** le ratio molaire hydrogène/1,2-dichloro-2,2-difluoroéthane est compris entre 0,2 et 20.

6. Procédé selon l'une quelconque des revendications précédentes **caractérisé en ce que** le ratio molaire hydrogène/1,2-dichloro-2,2-difluoroéthane est compris entre 0,5 et 10.

7. Procédé selon l'une quelconque des revendications précédentes **caractérisé en ce que** le ratio molaire hydrogène/1,2-dichloro-2,2-difluoroéthane est compris entre 1 et 6.

8. Procédé selon l'une quelconque des revendications précédentes **caractérisé en ce que** la pyrolyse est mise en oeuvre en présence d'un gaz inerte.

## Patentansprüche

1. Verfahren zur Herstellung von Vinylidenfluorid, wobei das Verfahren aus einer Pyrolyse von 1,2-Dichlor-2,2-difluorethan in Gegenwart von Wasserstoff und in Abwesenheit eines Katalysators bei einer Temperatur von größer oder gleich 400 °C besteht.

2. Verfahren nach Anspruch 1, **dadurch gekennzeichnet, dass** die Temperatur zwischen 400 und 1000 °C liegt.

3. Verfahren nach Anspruch 1 oder 2, **dadurch gekennzeichnet, dass** die Temperatur zwischen 450 und 750 °C liegt.

4. Verfahren nach einem der vorhergehenden Ansprüche, **dadurch gekennzeichnet, dass** die Temperatur zwischen 500 und 600 °C liegt.

5. Verfahren nach einem der vorhergehenden Ansprüche, **dadurch gekennzeichnet, dass** das Molverhältnis Wasserstoff/ 1,2-Dichlor-2,2-difluorethan zwischen 0,2 und 20 liegt.

6. Verfahren nach einem der vorhergehenden Ansprüche, **dadurch gekennzeichnet, dass** das Molverhältnis Wasserstoff/ 1,2-Dichlor-2,2-difluorethan zwischen 0,5 und 10 liegt.

7. Verfahren nach einem der vorhergehenden Ansprüche, **dadurch gekennzeichnet, dass** das Molverhältnis Wasserstoff/ 1,2-Dichlor-2,2-difluorethan zwischen 1 und 6 liegt.

8. Verfahren nach einem der vorhergehenden Ansprüche, **dadurch gekennzeichnet, dass** die Pyrolyse in Gegenwart eines Inertgases durchgeführt wird.

## Claims

1. A process for obtaining vinylidene fluoride, consisting in the pyrolysis of 1,2-dichloro-2,2-difluoroethane in the presence of hydrogen and in the absence of catalyst at a temperature of greater than or equal to 400°C.

2. The process as claimed in claim 1, **characterized in that** the temperature is between 400 and 1000°C.

3. The process as claimed in claim 1 or 2, **characterized in that** the temperature is between 450 and 750°C.

4. The process as claimed in any one of the preceding claims, **characterized in that** the temperature is between 500 and 600°C.

5. The process as claimed in any one of the preceding claims, **characterized in that** the hydrogen/1,2-dichloro-2,2-difluoroethane molar ratio is between 0.2 and 20.

6. The process as claimed in any one of the preceding claims, **characterized in that** the hydrogen/1,2-dichloro-2,2-difluoroethane molar ratio is between 0.5 and 10.

7. The process as claimed in any one of the preceding claims, **characterized in that** the hydrogen/1,2-dichloro-2,2-difluoroethane molar ratio is between 1 and 6.

8. The process as claimed in any one of the preceding claims, **characterized in that** pyrolysis is carried out in the presence of an inert gas.
